# EUROPÄISCHE PATENTSCHRIFT

(11) **EP 2 589 821 B1**
(45) Veröffentlichungstag und Bekanntmachung des Hinweises auf die Patenterteilung: **02.08.2017**
(21) Anmeldenummer: 12006613.9
(22) Anmeldetag: 20.09.2012
(51) Int. Cl.: F04F 5/20, A61B 17/88, B01F 13/00

(54) **Vorrichtung und Verfahren zur Erzeugung von Vakuum für Vakuumzementiersysteme**
Apparatus and method for the generation of vacuum for vacuum cementing systems
Dispositif et procédé de génération de vide pour système de cimentation sous vide

(30) Priorität: 03.11.2011 DE 102011117527
(43) Veröffentlichungstag der Anmeldung: 08.05.2013
(73) Patentinhaber: Heraeus Medical GmbH, 61273 Wehrheim (DE)
(72) Erfinder: Vogt, Sebastian, Dr., 99092 Erfurt (DE); Wüst, Edgar, 64823 Gross-Umstadt (DE)
(74) Vertreter: Schultheiss & Sterzel Patentanwälte PartG mbB

(56) Entgegenhaltungen:
- EP-A2- 2 269 718
- WO-A1-00/35506
- WO-A1-03/009766
- US-A1- 2010 091 606
- US-A1- 2010 262 152

## Beschreibung

Die Erfindung betrifft eine Vorrichtung zum Erzeugen eines Vakuums für eine Vakuumzementiervorrichtung, wobei die Vorrichtung eine Gaspatrone, einen Kanal und einen Anschluss für die Gaspatrone aufweist.

Die Erfindung betrifft auch ein Verfahren zum Erzeugen eines Unterdrucks in einem Vakuumzementiersystem.

PMMA-Knochenzemente sind seit Jahrzehnten bekannt und gehen auf die grundlegenden Arbeiten von Sir Charnley zurück (Charnley, J.: "Anchorage of the femoral head prosthesis of the shaft of the femur" J. Bone Joint Surg. 42 (1960) 28-30.). Der Grundaufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Bei dem Vermischen der Monomerkomponente mit der Pulverkomponente reagiert der Aktivator N,N-Dimethyl-p-toluidin mit dem Dibenzoylperoxid, das unter Bildung von Radikalen zerfällt. Die gebildeten Radikale initiieren die radikalische Polymerisation des Methylmethacrylats. Mit fortschreitender Polymerisation des Methylmethacrylats erhöht sich die Viskosität des Zementteigs bis der Zementteig erstarrt und damit ausgehärtet ist.

Polymethylmethacrylat-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird.

Im Vakuum gemischte Knochenzemente haben eine deutlich verringerte Porosität und zeigen daher verbesserte mechanische Eigenschaften. Es wurden eine Vielzahl von Vakuumzementiersystemen offen gelegt, von denen exemplarisch die folgenden genannt seien: US 5,624,184 A, US 4,671,263 A, US 4,973,168 A, US 5,100,241 A, WO99/67015, EP 1 020 167 A1, US 5,586,821 A, EP 1 016 452 A1, DE 36 40 279 A1, WO94/26403, EP 0 692 229 A1, EP 1 005 901 A1, US 5,344,232 A.

Aus der US 2010/0262152 A1 und der US 2010/0091606 A1 sind Zementiersysteme bekannt, bei denen ein Knochenzement in einer Spritze gemischt werden kann.

Die WO 03/009766 A1 offenbart eine Vakuumglocke für die Geburtshilfe, bei der ein Unterdruck durch ein ausströmendes Gas erzeugt wird.

Die WO 00/35506 A1 beschreibt ein Verfahren zum Mischen eines Knochenzements unter Vakuum.

Bei den gegenwärtig im Markt üblichen Vakuumzementiersystemen wird das erforderliche Vakuum über mit unsteriler Druckluft betriebene Vakuumpumpen erzeugt. Diese Schlauchsysteme sind schwer und stellen immer auch auf Grund ihrer Verlegung auf dem Fußboden eine Unfallgefahr dar. Daneben ist es auch möglich, durch zentrale Vakuumleitungssysteme Vakuum im Operationssaal bereit zu stellen. Dabei ergeben sich auf Grund der Verwendung von langen Schläuchen ebenfalls die Probleme der Unfallgefahr und auch der schlechten Handhabbarkeit. Ferner muss am Ort der Operation oder in der unmittelbaren Umgebung ein Vakuum erzeugt werden, wobei hierfür Druckluft und/oder Strom vorhanden sein muss. Dies ist nicht immer möglich, wenn eine Operation (OP) in weniger entwickelten Gegenden, an einer Unfallstelle, in provisorisch eingerichteten Notfalloperationssäälen, in Zelten oder in einem Feldlazarett durchgeführt werden soll.

Die Aufgabe der Erfindung besteht darin, die Nachteile des Stands der Technik zu überwinden. Insbesondere soll eine mobile und leichte Vorrichtung zur Erzeugung von Vakuum für Zementiersysteme bereitgestellt werden. Vorteilhaft wäre es auch, wenn die Vorrichtung ohne lange und schwere Schläuche auskommt und möglichst nicht an stationäre Druckluftsysteme, ein Stromnetz oder auch an Vakuumsysteme angeschlossen werden muss. Dem medizinischen Anwender im Operationssaal soll eine leicht zu handhabende, sichere Vorrichtung zur Verfügung gestellt werden. Ein Ziel ist auch, die Vorrichtung so einfach zu gestalten, dass diese aus kostengünstigen Materialien aufgebaut werden kann und dass dadurch eine wirtschaftliche Fertigung als Einmalartikel möglich ist.

Die Aufgabe der Erfindung besteht ferner darin, ein möglichst einfaches Verfahren zu entwickeln, das vom OP-Personal während einer OP mit geringstem Zeitaufwand zum Herstellen eines Knochenzements unter Vakuum eingesetzt werden kann.

Die Aufgaben der Erfindung und weitere nicht genannte Probleme werden dadurch gelöst, dass der Anschluss eine Öffnungseinrichtung zum Öffnen der Gaspatrone umfasst und die Gaspatrone über den Anschluss mit dem Kanal druckdicht verbindbar oder verbunden ist, so dass ein Gas aus der geöffneten Gaspatrone entlang des Kanals strömt, wobei der Kanal den Anschluss mit der Umgebung der Vorrichtung verbindet und der Kanal zumindest ein T-Stück mit einem Vakuumanschluss für die Vakuumzentiervorrichtung umfasst, wobei das T-Stück als Venturi-Düse ausgebildet ist, so dass das durch den Kanal strömende Gas aus der geöffneten Gaspatrone am Vakuumanschluss einen Unterdruck erzeugt, wobei die Venturi-Düse in einen Ausströmkanal mit einem vergrößerten Durchmesser mündet, der gasdurchlässig mit der Umgebung der Vorrichtung verbunden ist.

Durch diese Merkmale wird eine Vorrichtung bereitgestellt, die ohne Kabel und ohne andere Leitungen auskommt und gleichzeitig in der Lage ist, zumindest kurzzeitig ein Vakuum zum Mischen eines medizinischen Zements bereitzustellen.

Unter einem Vakuum wird vorliegend auch Grobvakuum, das heißt auch ein kleinerer Unterdruck verstanden. Vorzugsweise wird unter einem Vakuum beziehungsweise einem Unterdruck ein Gasdruck von kleiner oder gleich 300 mbar verstanden, besonders bevorzugt von kleiner oder gleich 80 mbar.

Bei einer erfindungsgemäßen Vorrichtung kann vorgesehen sein, dass im Kanal zumindest ein bedienbares Absperrelement angeordnet ist, mit dem der Gasstrom aus der geöffneten Gaspatrone durch den Kanal unterbrechbar und/oder einstellbar ist, wobei insbesondere das bedienbare Absperrelement ein Rückstellelement umfasst, das vorzugsweise ein Federelement umfasst, besonders bevorzugt ein Federelement aus Metall.

Über ein Absperrelement, wie beispielsweise ein Ventil kann die Vorrichtung leichter gesteuert werden und bietet so mehr Bedienkomfort. Es können auch mehrere Vakuumzementiersysteme nacheinander mit einer solchen Vorrichtung mit einem Vakuum beaufschlagt werden, sofern die Gaspatrone noch ausreichend komprimiertes Gas enthält.

Dabei kann vorgesehen sein, dass das Absperrelement ein Ventil oder die Absperrelemente Ventile sind, vorzugsweise zumindest ein Drehventil, ein Kugelventil, ein Quetschventil, ein Stiftventil und/oder ein Schieber.

Solche Ventile sind einfach und kostengünstig aufbaubar und dadurch zur Umsetzung einer kostengünstigen Vorrichtung besonders geeignet.

Dabei kann wiederum vorgesehen sein, dass das Ventil ein konisch geformter, zumindest bereichsweise rotationssymmetrischer Körper ist, der in einer zur Form des Ventils passenden konischen Öffnung in der Vorrichtung drehbar gelagert ist, wobei die Außenwände des Ventils bis auf die Öffnungen des Kanals dicht mit den Wänden der konischen Öffnung in der Vorrichtung in jeder Position des Ventils abschließen, so dass die Verbindungen zumindest zu dem Kanal dicht sind.

Ein solches Ventil kann beispielsweise über einen Keil in die konische Öffnung der Vorrichtung eingepresst werden, so dass mit diesem sehr einfachen Aufbau ein Presssitz des Ventils erreicht wird. Solche Ventile lassen sich in einfacher Weise durch Spritzgießtechnik aus Kunststoff fertigen.

Eine weitere besonders bevorzugte Ausgestaltung der Erfindung kann vorsehen, dass der Anschluss ein Innengewinde zur Aufnahme einer Gaspatrone mit einem Außengewinde umfasst.

Über ein Gewinde lässt sich in einfacher Weise eine große Kraft auf eine Sollbruchstelle an der Gaspatrone ausüben. Zudem haben viele handelsübliche Gaspatronen bereits ein solches Gewinde, so dass diese für eine erfindungsgemäße Vorrichtung verwendbar sind.

Ferner kann vorgesehen sein, dass der Anschluss ein Dichtungsmittel, vorzugsweise eine Gummidichtung zum Abdichten der Verbindung der Gaspatrone mit dem Kanal umfasst.

Hierdurch kann einer möglichen Fehlfunktion durch nicht oder zu wenig in den Kanal strömendes Gas vermieden werden. Zudem wird die Effizienz der Vorrichtung gesteigert, wenn der Anschluss durch das Dichtungsmittel bis zu möglichst hohem Druck dicht bleibt.

Gemäß einer weiteren Ausgestaltung der Erfindung kann vorgesehen sein, dass die Öffnungseinrichtung einen Dorn umfasst, vorzugsweise einen Hohldorn, der ein Ende des Kanals bildet.

Dies stellt eine einfache und auch kostengünstig zu realisierende Möglichkeit dar, eine Öffnungseinrichtung zu realisieren. Insbesondere im Zusammenhang mit einem Gewinde an der Gaspatrone und auch ggfs. einem Drehgriff, der an der Gaspatrone befestigt ist, kann so die Gaspatrone leicht geöffnet werden.

Dabei kann vorgesehen sein, dass die Öffnungseinrichtung eine bedienbare Presseinrichtung umfasst, mit der die Gaspatrone auf den Dorn pressbar ist, insbesondere im Bereich einer Membran als Sollbruchstelle der Gaspatrone, wobei die Gaspatrone dadurch zu öffnen ist und wobei vorzugsweise die Presseinrichtung drehbar ist.

Eine weitere besonders bevorzugte Ausgestaltung der Erfindung kann dadurch realisiert werden, dass die Vorrichtung die Gaspatrone umfasst, wobei die Gaspatrone derart im Bereich der Öffnungseinrichtung in die Vorrichtung eingebaut ist, dass sie durch ein Bedienen der Öffnungseinrichtung zu öffnen ist, wobei die Gaspatrone vorzugsweise ein Außengewinde umfasst, das in das Innengewinde des Anschlusses greift.

Eine bereits eingebaute Gaspatrone vereinfacht die Anwendung, was im zuweilen hektischen OP-Betrieb ein großer Vorteil ist.

Besonders bevorzugte Ausgestaltungen der Erfindung ergeben sich, wenn vorgesehen ist, dass die Venturi-Düse durch eine Verjüngung des Kanals gebildet ist und der Vakuumanschluss im Bereich dieser Verjüngung mit dem Kanal verbunden ist, wobei vorzugsweise eine Laval-Düse gebildet ist.

Die Verjüngung sorgt für eine Beschleunigung der durchströmenden Gase und damit für einen geringeren Gasdruck senkrecht dazu, das heißt ein besseres Vakuum am Vakuumanschluss.

Es kann auch vorgesehen sein, dass die Vorrichtung zumindest eine Bedieneinrichtung umfasst, mit der die Öffnungseinrichtung und/oder das Absperrelement bedienbar ist oder sind, wobei die Bedieneinrichtung als Abzug, als Taste oder als Pedal ausgebildet ist und wobei die Bedieneinrichtung vorzugsweise eine Rückstelleinrichtung, insbesondere mit einer Metallfeder umfasst.

Diese Bedieneinrichtungen sind für den OP-Betrieb besonders geeignet, da hierdurch keine aufwendigen Maßnahmen zum Bedienen der Vorrichtung notwendig sind.

Auch kann vorgesehen sein, dass an der Unterseite der Vorrichtung ein Griff zum Halten der Vorrichtung angeordnet ist, wobei vorzugsweise die Bedieneinrichtung ein Abzug oder eine Taste im Bereich des Griffs der Vorrichtung ist, so dass die Vorrichtung einhändig bedienbar ist.

Hierdurch kann die Vorrichtung in der Hand gehalten werden, um eine manuelle Bedienung zu erleichtern.

Alternativ dazu kann vorgesehen sein, dass die Vorrichtung mit einem ebenen Boden zum Aufstellen auf einem ebenen Untergrund ausgebildet ist, wobei vorzugsweise die Bedieneinrichtung ein Pedal oder eine Taste auf der Oberseite der Vorrichtung ist.

Diese Variante ermöglicht das Abstellen der Vorrichtung auf dem Boden eines Operationssaals. Die Hände des Anwenders bleiben dann frei, um andere Tätigkeiten durchzuführen.

Besonders bevorzugt kann vorgesehen sein, dass die Vorrichtung im Wesentlichen aus Kunststoff besteht, vorzugsweise bis auf die Öffnungseinrichtung, die Gaspatrone und/oder das Rückstellelement, das Rückschlagventil oder die Rückstellelemente aus Kunststoff besteht.

Die Verwendung von Kunststoff stellt sicher, dass die Herstellungskosten der Vorrichtung gering sind. Zudem sind einige Kunststoffe für Medizinprodukte besonders geeignet. Eine Fertigung aus preisgünstigen Kunststoffspritzgießteilen, die mit geringstem Montageaufwand zusammengesetzt werden können, ist also besonders bevorzugt.

Ferner kann vorgesehen sein, dass die Vorrichtung eine Masse von weniger als 10 kg hat, vorzugsweise zwischen 0,1 kg und 5 kg, besonders bevorzugt zwischen 0,5 kg und 3 kg.

Die geringe Masse der Vorrichtung vereinfacht die Handhabbarkeit der Vorrichtung.

Gemäß einer weiteren besonders bevorzugten Ausgestaltung der Erfindung kann vorgesehen sein, dass der Kanal an der dünnsten Stelle eine Querschnittsfläche von weniger als 10 mm² hat, insbesondere zwischen 0,1 mm² und 5 mm² und/oder der Kanal einen schmalen Spalt mit einer Breite von weniger als 1 mm bildet oder eine schlanke Röhre mit einem Durchmesser von weniger als 1 mm ist.

Diese Querschnittsflächen, Breiten und Durchmesser des Kanals sorgen dafür, dass das Gas aus gebräuchlichen Gasdruckpatronen ausreichend lange durch die Vorrichtung strömt, also der Unterdruck beziehungsweise das Vakuum über eine ausreichend lange Zeit zum Mischen eines medizinischen Zements erhalten bleibt.

Es kann auch vorgesehen sein, dass die Geometrie und der Strömungswiderstand des Kanals derart an die Kapazität der Gaspatrone angepasst sind, dass das Gas aus der geöffneten Gaspatrone zumindest 5 s aus der Gaspatrone durch den Kanal strömen kann, bevor die Gaspatrone kein komprimiertes Gas mehr enthält, vorzugsweise zwischen 10 s und 100 s, besonders bevorzugt zwischen 20 s und 60 s, ganz besonders bevorzugt zwischen 25 s und 45 s.

Diese Zeiträume sind zum Mischen eines medizinischen Zements besonders geeignet.

Eine weitere erfindungsgemäße Ausgestaltung der Vorrichtung kann vorsehen, dass im Vakuumanschluss ein Rückschlagventil angeordnet ist, wobei das Rückschlagventil durch den durch die Venturi-Düse erzeugten Unterdruck zu öffnen ist und den Vakuumanschluss verschließt, wenn kein oder sehr wenig Gas aus der geöffneten Gaspatrone durch den Kanal strömt und wobei vorzugsweise das Rückschlagventil ein Rückstellelement umfasst.

Durch diese Maßnahme kann besonders gut auf eine Absperreinrichtung verzichtet werden. Zudem wird die Vorrichtung so abgedichtet und dadurch unanfälliger für Störungen. Vor allem aber kann dadurch das Vakuum am angeschlossenen Vakuumzementiersystem erhalten bleiben, auch wenn die Vorrichtung nicht mehr betätigt wird.

Es ist vorgesehen, dass die Venturi-Düse in einen Ausströmkanal mit einem vergrößerten Durchmesser mündet, der gasdurchlässig mit der Umgebung der Vorrichtung verbunden ist, wobei im Ausströmkanal vorzugsweise ein Schalldämpfer angeordnet ist, besonders bevorzugt im Bereich der Mündung zur Umgebung der Vorrichtung.

Dies verbessert die Strömungsgeschwindigkeit des Gases und damit das erzeugte Vakuum. Der Schalldämpfer soll störenden Geräuschen durch das strömende Gas vorbeugen.

Es kann ferner vorgesehen sein, dass die Vorrichtung über den Vakuumanschluss an ein Vakuumzementiersystem zur Herstellung eines medizinischen Knochenzements angeschlossen ist oder anschließbar ist.

Die Aufgabe der Erfindung wird auch gelöst durch ein Verfahren zum Erzeugen eines Unterdrucks in einem Vakuumzementiersystem, insbesondere mit einer solchen Vorrichtung, umfassend die folgenden Schritte:
Öffnen einer Gaspatrone,
Durchströmen des Gases aus der Gaspatrone durch eine Venturi-Düse, wodurch der Unterdruck erzeugt wird und
Anlegen des Unterdrucks an das Vakuumzementiersystem.

Dabei kann vorgesehen sein, dass nach dem Öffnen der Gaspatrone das Absperrelement geöffnet wird und nach dem Öffnen des Absperrelements das Gas durch die Venturi-Düse strömt.

Hierdurch wird das Verfahren besser steuerbar.

Es kann auch vorgesehen sein, dass ein medizinischer Zement, insbesondere ein medizinischer Knochenzement unter Einwirkung des Unterdrucks im Vakuumzementiersystem gemischt wird.

De Erfindung liegt die überraschende Erkenntnis zugrunde, dass es mit einer einfachen und von externer Versorgung unabhängigen Vorrichtung, die ein komprimiertes Gas verwendet, gelingen kann, ein Vakuum zum Mischen eines medizinischen Zements zu erzeugen. Es wird genutzt, dass ein komprimiertes Gas ausströmt und dabei einen Unterdruck erzeugt, indem im Strömungsweg eine Venturi-Düse angeordnet ist. Durch die Schaltbarkeit des Gasstroms kann bei Verwendung eines Bedienelements, mit dem eine Absperreinrichtung oder eine Öffnungseinrichtung für eine Gaspatrone bedient werden kann, auch der Unterdruck geschaltet werden. Durch die erfindungsgemäßen Maßnahmen gelingt es, für einen kurzen Zeitraum einen Unterdruck bereitzustellen, der zum Mischen eines medizinischen Zements geeignet ist. Der kurze Zeitraum reicht aus, um den medizinischen Zement unter Unterdruck zu mischen.

Die erfindungsgemäße Vorrichtung kann auch ohne Elektromotoren und damit ohne Strom oder potentiell umweltgefährdende Batterien und Akkumulatoren betrieben werden. Eine Anwendung ist auch unter schwierigen Bedingungen ohne den Zugang zu weiteren Ressourcen ohne weiteres möglich.

Eine beispielhafte erfindungsgemäße Vorrichtung zur Erzeugung von Vakuum ist aufgebaut aus
a) einem Druckkörper,
b) einer zylinderförmigen Öffnung im Druckkörper mit Innengewinde zur Aufnahme der Gaspatrone mit Außengewinde,
c) einem Dichtungsmittel am Boden der zylinderförmigen Öffnung,
d) einem hohlen Stechdorn, der unterhalb des Dichtungsmittels am Boden der zylinderförmigen Öffnung angeordnet ist, und durch eine Öffnung der Dichtungsscheibe nach außen ragt,
e) einer mit komprimiertem Gas befüllten Gaspatrone, die über ihr Außengewinde in das Innengewinde der zylinderförmigen Öffnung geschraubt ist,
f) einem um seine Achse drehbaren Öffnungsmittel zum Drehen der Gaspatrone, das mit der Gaspatrone nicht drehbar verbunden ist,
g) einer Öffnung im Druckkörper zur Aufnahme eines schaltbaren Absperrelements, wobei diese Öffnung durch einen Kanal gasdurchlässig mit der zylinderförmigen Öffnung mit Innengewinde verbunden ist,
h) einem Kanal der die Öffnung mit einer Düse gasdurchlässig verbindet, wobei die Düse, in einem Ausströmkanal mündet, der gasdurchlässig mit einem Kanal mit der Außenseite des Druckkörpers verbunden ist,
i) einem schaltbaren Absperrelement, das den Kanal gasdurchlässig verbinden oder gasundurchlässig trennen kann und das in der Öffnung angeordnet ist, wobei das schaltbare Absperrelement mit einem Rückstellelement verbunden ist, und
j) einem Ausströmkanal, der mit dem Kanal verbunden ist und in den die Düse mündet, wobei der Ausströmkanal vorzugsweise als Laval-Düse ausgebildet und die Öffnung der Düse in Richtung der konischen Verjüngung der Lavaldüse ausgerichtet ist und dieser Ausströmkanal gasdurchlässig mit der umgebenden Atmosphäre verbunden ist.

Eine alternative erfindungsgemäße Vorrichtung, kann aufgebaut sein aus
a) einem Druckkörper,
b) einer zylinderförmigen Öffnung im Druckkörper mit Innengewinde zur Aufnahme der Gaspatrone mit Außengewinde,
c) einem Dichtungsmittel am Boden der zylinderförmigen Öffnung,
d) einem hohlen Stechdorn, der unterhalb des Dichtungsmittels am Boden der zylinderförmigen Öffnung angeordnet ist, und durch eine Öffnung der Dichtungsscheibe nach außen ragt,
e) einer Gaspatrone, die über ihr Außengewinde in das Innengewinde der zylinderförmigen Öffnung geschraubt ist,
f) einem um seine Achse drehbaren Öffnungsmittel zum Drehen der Gaspatrone, das mit der Gaspatrone drehfest verbunden ist,
g) einer Öffnung im Druckkörper zur Aufnahme eines Drehventils, wobei diese Öffnung durch einen ersten Kanal gasdurchlässig mit der zylinderförmigen Öffnung mit Innengewinde verbunden ist,
h) einem zweiten Kanal der die Öffnung mit einer Düse gasdurchlässig verbindet, wobei die Düse, in einen Ausströmkanal mündet, der gasdurchlässig mit einem dritten Kanal mit der Außenseite des Druckkörpers verbunden ist,
i) einem Drehventil, das den ersten Kanal mit dem zweiten Kanal in der Position gasdurchlässig verbinden oder gasundurchlässig trennen kann, das in der Öffnung angeordnet ist, wobei das Drehventil mit einem Rückstellelement verbunden ist, und
j) einem Ausströmkanal, der mit dem zweiten Kanal verbunden ist und in den die Düse mündet, wobei der Ausströmkanal als Laval-Düse ausgebildet und die Öffnung der Düse in Richtung der konischen Verjüngung der Lavaldüse ausgerichtet ist und dieser Ausströmkanal gasdurchlässig mit der umgebenden Atmosphäre verbunden ist.

Die Druckkörper bilden ein Druckfestes Gehäuse für die Vorrichtung.

Besonders vorteilhaft können Gaspatronen die verflüssigtes Gas, wie flüssiges Kohlendioxid enthalten, in der Vorrichtung eingesetzt werden. Daneben sind auch alle anderen nicht toxischen Gase verwendbar.

Der Ausströmkanal kann gegebenenfalls auch ein schalldämpfendes Element, wie Porenschreiben, Filzscheiben oder Gewebescheiben enthalten, um eine Geräuschdämpfung des ausströmenden Gases zu erreichen.

Das Absperrelement kann als Drehventil, Kugelventil, Quetschventil, Stiftventil und Schieber ausgebildet sein, wobei das Drehventil besonders bevorzugt wird. Das Absperrelement kann mit einem Betätigungselement, wie einem Abzug oder einer Fußtaste oder einer Drucktaste kraftschlüssig oder formschlüssig verbunden sein. Dadurch ist eine manuelle Betätigung oder auch eine Fußbetätigung des Absperrelements möglich.

Das Rückstellelement kann als Spiralfeder oder als Blattfeder ausgebildet sein und besteht bevorzugt aus Federstahl. Die Gaspatrone ist ebenfalls aus Metall gefertigt und besteht bevorzugt aus Stahl oder aus einer Aluminiumlegierung.

Die erfindungsgemäße Vorrichtung kann in einem Gehäuse angeordnet sein. Dabei kann das Betätigungselement als Fußtaste oder als Abzug ausgebildet sein und aus dem Gehäuse herausragen oder so in einer Aussparung im Gehäuse angeordnet sein, dass eine manuelle Bedienung oder auch eine Fußbedienung des Betätigungselements möglich ist.

Die Vorrichtung kann in unsterilem oder auch in sterilem Zustand dem medizinischen Anwender zur Verfügung gestellt werden. Die Vorrichtung mit dem in der Gaspatrone enthaltenem Gas kann vorteilhaft durch Gammasterilisation sterilisiert werden. Dadurch kann auch das sterile Gas problemlos in die Atmosphäre des Operationssaals abgegeben werden, ohne eine mikrobielle Kontamination zu verursachen.

Zu der erfindungsgemäßen Vorrichtung gehört auch ein Verfahren zum Erzeugen von Vakuum mit dieser Vorrichtung. Dieses erfindungsgemäße Verfahren kann durch folgende nacheinander ablaufende Schritte charakterisiert sein:
a) Drehen des Öffnungsmittels, bis die Gaspatrone so gegen den Stechdorn bewegt wird, dass eine Membran der Gaspatrone durchstochen wird,
b) Ausströmen des komprimierten Gases bis zum Absperrelement,
c) Öffnen des Absperrelements durch Betätigung der Fußtaste oder des Abzugs, wobei das komprimierte Gas durch den Kanal zur Düse fließt, aus der Düse in den Ausströmkanal sich entspannt und danach durch den Ausströmkanal an die umgebende Atmosphäre abgegeben wird,
d) wobei sich an oder um die Düse ein Unterdruck ausbildet und dieser Unterdruck sich bis zum Vakuumanschluss ausbreitet.

Im Folgenden werden Ausführungsbeispiele der Erfindung anhand von sechs schematisch dargestellten Figuren erläutert, ohne jedoch dabei die Erfindung zu beschränken. Dabei zeigt:
- Figur 1:: eine schematische Seitenansicht einer erfindungsgemäßen Vorrichtung zum Erzeugen eines Vakuums für eine Vakuumzementiervorrichtung mit einem Handgriff;
- Figur 2:: eine schematische perspektivische Ansicht einer alternativen erfindungsgemäßen Vorrichtung zum Erzeugen eines Vakuums für eine Vakuumzementiervorrichtung zum Aufstellen auf dem Boden;
- Figur 3:: eine schematische Frontalansicht der erfindungsgemäßen Vorrichtung nach Figur 2;
- Figur 4:: eine schematische Querschnittansicht einer dritten erfindungsgemäßen Vorrichtung zum Erzeugen eines Vakuums für eine Vakuumzementiervorrichtung;
- Figur 5:: eine schematische Querschnittansicht einer vierten erfindungsgemäßen Vorrichtung zum Erzeugen eines Vakuums für eine Vakuumzementiervorrichtung; und
- Figur 6:: eine schematische perspektivische Ansicht eines Absperrelements für eine erfindungsgemäße Vorrichtung.

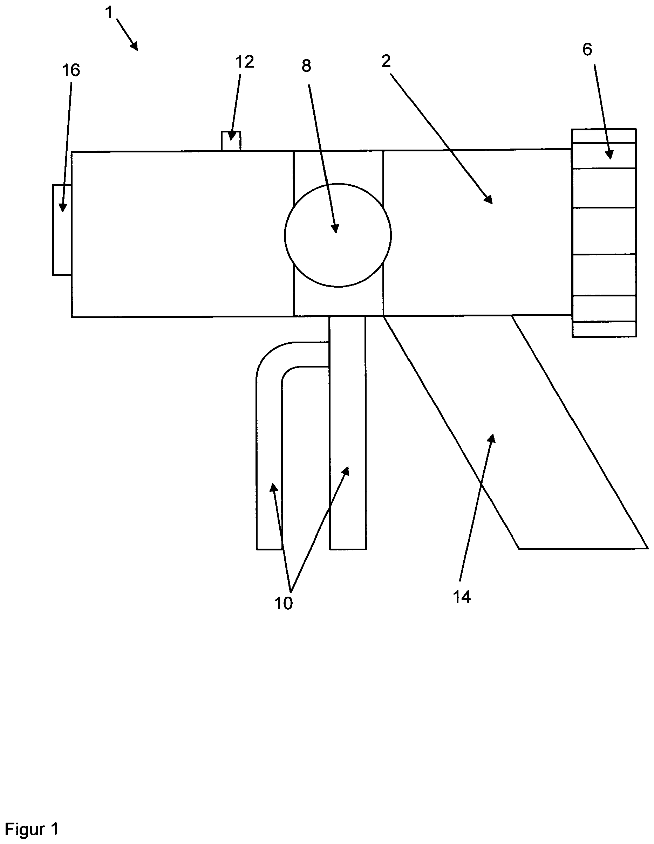

Figur 1 zeigt eine schematische Querschnittansicht einer erfindungsgemäßen Vorrichtung 1 zum Erzeugen eines Vakuums für eine Vakuumzementiervorrichtung. Die Vorrichtung 1 umfasst ein Gehäuse 2, in dem eine Gaspatrone (nicht gezeigt) angeordnet ist. Die Rückseite der Vorrichtung 1 ist mit einem Schraubdeckel 6 verschlossen, mit dem die Gaspatrone im Inneren der Vorrichtung verschoben und auf einen Dorn oder eine Klinge gepresst werden kann, so dass sich die Gaspatrone öffnet.

In der Mitte der Vorrichtung 1 ist ein drehbares Ventil 8 angeordnet, mit dem ein im Inneren der Vorrichtung 1 liegender Kanal (nicht gezeigt) geöffnet und geschlossen werden kann. Das drehbare Ventil 8 ist konisch geformt und umfasst eine Durchführung, die einen vorderen Teil und einen hinteren Teil des Kanals bei geeigneter Stellung des Ventils 8 verbinden kann und so den Kanal öffnet. Das drehbare Ventil 8 ist leicht konisch geformt und sitzt im Presssitz in einer Öffnung im Gehäuse 2 in der Vorrichtung 1, so dass eine gasdichte Verbindung der Durchführung mit dem Kanal beziehungsweise ein gasdichter Verschluss des Kanals möglich ist.

Das drehbare Ventil 8 kann über einen Abzug 10 bedient werden. Das drehbare Ventil 8 kann mit einer federnden Rückstelleinrichtung verbunden sein, die eine geschlossene Stellung des Ventils bewirkt, wenn keine Kraft auf den Abzug 10 wirkt. Auf der Oberseite der Vorrichtung 1 ist ein Vakuumanschluss 12 angeordnet, der im Inneren der Vorrichtung 1 eine Zuleitung umfasst, die senkrecht auf den Kanal trifft, wobei die Verbindung der Zuleitung mit dem Kanal eine Venturi-Düse bildet, so dass bei einer Strömung durch den Kanal ein Unterdruck am Vakuumanschluss 12 erzeugt wird. An den Vakuumanschluss 12 ist eine Vakuumzementiervorrichtung (nicht gezeigt) anschließbar oder angeschlossen.

Auf der Unterseite der Vorrichtung 1 ist ein Griff 14 angeordnet, so dass die Vorrichtung nach Art einer Pistole gehalten werden kann und der Abzug 10 mit der gleichen Hand bedient werden kann, mit der die Vorrichtung 1 am Griff 14 gehalten wird. Der Kanal in der Vorrichtung 1 mündet auf der Vorderseite in einen Gasauslass 16, durch den das Gas aus der Gaspatrone bei geöffnetem Ventil 8 aus der Vorrichtung ausströmen kann.

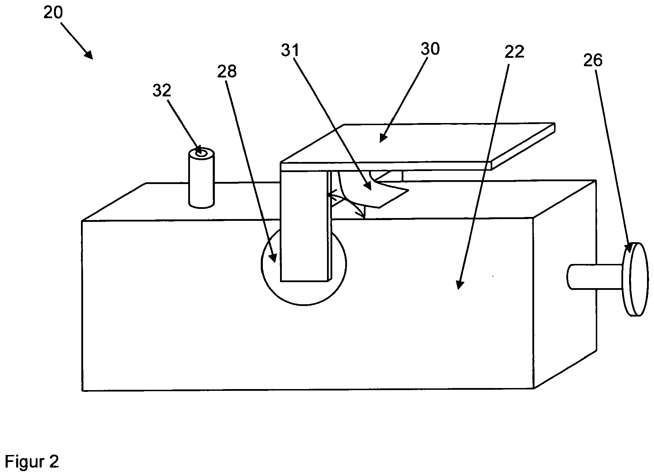

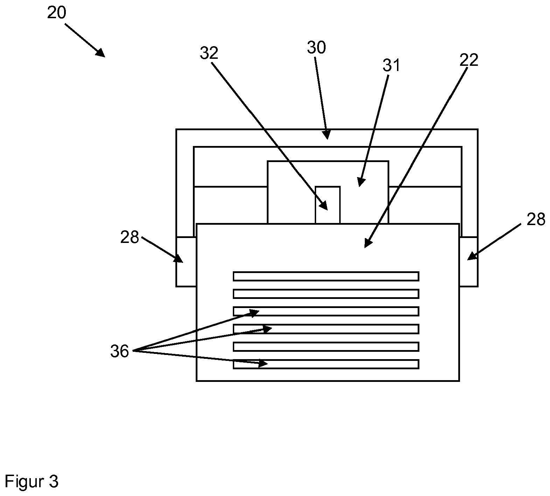

Die Figuren 2 und 3 zeigen eine alternative erfindungsgemäße Vorrichtung 20 zum Erzeugen eines Vakuums für eine Vakuumzementiervorrichtung, die mit dem Fuß bedienbar ist und auf dem Boden eines OP-Saals gestellt werden kann. Figur 2 zeigt die Vorrichtung in schematischer perspektivischer Ansicht und Figur 3 in schematischer Frontalansicht. In einem Gehäuse 22 der Vorrichtung 20 ist im Inneren ein Kanal mit einer Venturi-Düse (nicht gezeigt) und eine Druckgaspatrone (nicht gezeigt) angeordnet, analog der Ausführung nach Figur 1.

Am hinteren Ende der Vorrichtung 20 ist ein Drehgriff 26 angeordnet, der in die Vorrichtung 20 hineingedreht werden kann und der dabei die Druckgaspatrone im Inneren der Vorrichtung 20 öffnet.

Ein drehbares Ventil 28 reicht durch die Vorrichtung 20 und kann je nach Stellung den Kanal im Inneren der Vorrichtung 20 verschließen oder öffnen. Das Ventil ist über eine Fußtaste 30 auf der Oberseite der Vorrichtung 20 bedienbar. Auf der Unterseite der Fußtaste 30 ist eine Blattfeder als Rückstellfeder 31 für die Fußtaste angeordnet. So wird ein Pedal auf der Oberseite der Vorrichtung 20 gebildet, mit dem das Ventil 28 bedienbar ist, wobei der Kanal mit dem Fuß zu öffnen und zu schließen ist. Die Rückstellfeder 31 bewirkt, dass die Fußtaste 30 in der gezeigten Stellung verbleibt, wenn die Fußtaste 31 nicht heruntergedrückt wird. In der heruntergedrückten Stellung (nicht gezeigt), ist das Ventil 28 geöffnet und in der gezeigten unbelasteten Stellung ist das Ventil 28 geschlossen.

Auf der Oberseite der Vorrichtung 20 ist ein Vakuumanschluss 32 in Form eines Rohrs angeordnet. Der Vakuumanschluss 32 kann eine übliche Schnellsteckverbindung für Vakuumanschlüsse umfassen. Über den Vakuumanschluss 32 kann eine Vakuumzementiervorrichtung (nicht gezeigt) angeschlossen sein. Auf der Vorderseite der Vorrichtung 20 sind Schlitze eines Gasauslasses 36 angeordnet, in die der Kanal mündet und durch die das Gas aus dem Kanal in die Umgebung strömen kann.

Um die Vorrichtung 20 zu nutzen, wird zunächst der Drehgriff 26 bedient. Dabei wird die im Inneren der Vorrichtung 20 angeordnete Druckgaspatrone geöffnet. Statt des Drehgriffs 26 könnte auch ein Druckknopf an gleicher Stelle angeordnet sein, der bei einer Bedienung desselben die Druckgaspatrone öffnet. So könnte auch das Öffnen der Druckgaspatrone mit dem Fuß, beispielsweise mit einem leichten Tritt geöffnet werden. Ebenso kann auch der Druckknopf über ein Pedal sogar auch über die Fußtaste 30 bedient werden.

Wenn die Druckgaspatrone geöffnet wurde, strömt das Gas aus der Patrone in den Kanal, wird aber von dem geschlossenen Ventil 28 gestoppt. Durch Bedienen des Ventils 28 mit der Fußtaste 30 kann das Ventil 28 geöffnet werden und somit eine Strömung durch den Kanal erzeugt werden. Durch die Venturi-Düse im Inneren der Vorrichtung 20 wird dadurch ein Vakuum beziehungsweise ein Unterdruck am Vakuumanschluss 32 erzeugt. Dieses Vakuum wird an eine Vakuumzementiervorrichtung angelegt, so dass dort ein medizinischer Zement unter Vakuum gemischt werden kann.

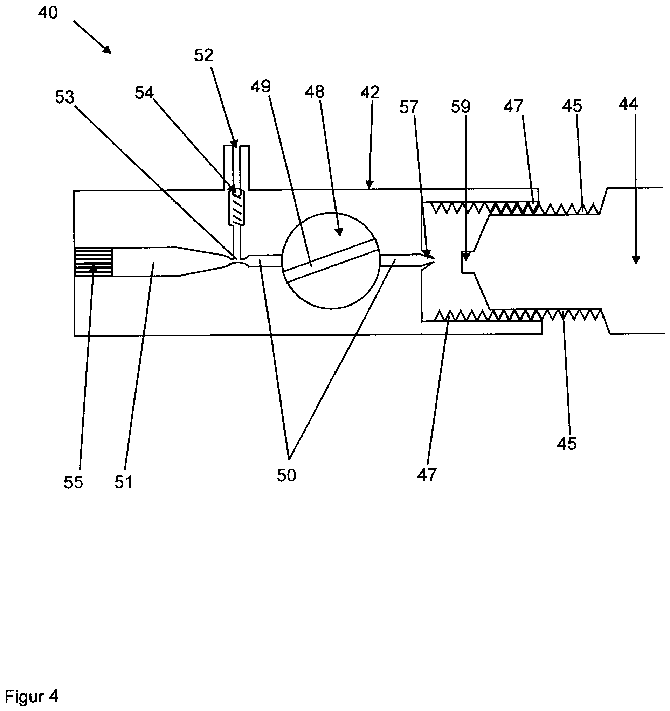

Figur 4 zeigt eine schematische Querschnittansicht einer erfindungsgemäßen Vorrichtung 40 zum Erzeugen eines Vakuums beziehungsweise eines Unterdrucks. Ein Vakuumzementiersystem wird mit dem Vakuum beziehungsweise dem Unterdruck beaufschlagt, um einen medizinischen Zement, insbesondere einen Knochenzement bei Unterdruck beziehungsweise im Vakuum zu mischen.

Die Vorrichtung 40 ist aus Kunststoff mit einem Spritzgießverfahren hergestellt. Das zentrale Teil bildet ein Gehäuse 42, das weitgehend massiv aufgebaut ist. Im hinteren Teil der Vorrichtung (in Figur 4 rechts) ist eine Gaspatrone 44 angeordnet, die mit einem unter Druck stehenden Gas gefüllt ist. Die Gaspatrone 44 kann auch mit einem verflüssigten Gas gefüllt sein, das sich unter Normaldruck in den gasförmigen Zustand umwandelt. Die Gaspatrone 44 umfasst ein Außengewinde 45, das in ein Innengewinde 47 in der Vorrichtung 40 greifen kann. Das Innengewinde 47 ist in einer Öffnung im hinteren Teil der Vorrichtung 40 angeordnet, die zum Einsetzen der Gaspatrone 44 vorgesehen ist.

Ein Ventil 48 ist drehbar im Presssitz in einer dafür vorgesehenen Ausnehmung Vorrichtung 40 angeordnet. In dem drehbaren Ventil 48 ist eine Durchführung 49 in Form einer zylindrischen Röhre angeordnet. Die Durchführung 49 ist dazu geeignet, einen Kanal 50 im Inneren der Vorrichtung 40 je nach Stellung des Ventils 48 zu öffnen oder zu schließen. Figur 4 zeigt die geschlossene Stellung des Ventils 48. Der Kanal 50 ist im vorderen Teil der Vorrichtung 40 (in Figur 4 links) als Ausströmkanal 51 mit größerem Querschnitt ausgebildet. Der Kanal 50 und der Ausströmkanal 51 sind zylindrische Röhren mit variablem Durchmesser oder Spalte mit variabler Breite.

Auf der Oberseite der Vorrichtung 40 ist ein Vakuumanschluss 52 angeordnet, der an ein Vakuumzementiersystem (nicht gezeigt) anschließbar ist oder angeschlossen ist. An einer Verengung des Kanals 50 trifft eine Zuleitung des Vakuumanschlusses 52 auf den Kanal 50 und ist dort mit diesem Verbunden. Der Kanal 50 und die Zuleitung des Vakuumanschlusses 52 bilden dort ein T-Stück 53. Der Kanal 50 bildet dabei eine lineare Durchführung auf die die Zuleitung senkrecht trifft. Durch die Verengung des Kanals 50 wird eine effiziente Venturi-Düse 53 durch das T-Stück 53 gebildet. Bei geeigneter Geometrie des Kanals 50 im Bereich des T-Stücks 53, kann die Venturi-Düse 53 auch als Laval-Düse ausgebildet sein.

In der Zuleitung zum Vakuumanschluss 52 befindet sich ein Rückschlagventil 54, das durch eine Kugel und eine Feder in einem erweiterten Bereich der Zuleitung zum Vakuumanschluss 52 realisiert wird. Die Kugel kann die Öffnung aus dem erweiterten Bereich heraus abschließen und wird von der Feder in dieser Position gehalten. Sobald durch die Venturi-Düse 53 ein Unterdruck erzeugt wird, wird das Rückschlagventil 54 nach unten gezogen und der Unterdruck am Vakuumanschluss 52 angelegt. Das Rückschlagventil 54 dient der Abdichtung der Vorrichtung 40, so dass diese auch ohne das Ventil 48 betrieben werden könnte. Es muss dann nur die Gaspatrone 44 geöffnet werden, um den Unterdruck am Vakuumanschluss 52 zu erzeugen. Zudem bleibt das Vakuum in dem Vakuumzementiersystem durch das Rückschlagventil 54 erhalten, auch wenn kein Gas mehr durch den Kanal 50 strömt. Sobald die Druckgaspatrone 44 geleert ist, liegt kein Unterdruck mehr an und die Vorrichtung 40 ist verbraucht oder muss mit einer neuen Druckgaspatrone 44 geladen werden.

Wenn die Vorrichtung 40 ein Ventil 48 umfasst, kann grundsätzlich auch auf das Rückschlagventil 54 verzichtet werden.

In der Mündung des Ausströmkanals 51 zur Umgebung der Vorrichtung 40 ist ein Schalldämpfer 55 angeordnet, der die beim Strömen des Gases durch den Kanal 50, 51 erzeugten Schallwellen dämpfen soll. Der Schalldämpfer 55 dient also lediglich dazu eine geräuschärmere Vorrichtung 40 bereitzustellen und ist für das Grundprinzip der Vorrichtung 40 unerheblich. Am hinteren Ende der Vorrichtung 40 mündet der Kanal 50 in einen Hohldorn 57, der dazu ausgelegt ist, eine dafür vorgesehene Stelle, insbesondere eine Membran am Kopf 59 der Gaspatrone 44 zu durchstechen.

Wenn also die Gaspatrone 44 in die Öffnung am hinteren Ende der Vorrichtung 40 geschraubt wird, wird die Gaspatrone 44 durch den Hohldorn 57 geöffnet. Das Gas strömt durch den Hohldorn 57 in den Kanal 50. Damit das komprimierte Gas aus der Gaspatrone 44 nicht an dem Hohldorn 57 vorbei entweicht, ist vorzugsweise eine Dichtung an dem Anschluss für die Gaspatrone 44 vorgesehen.

Das Ventil 48 befindet sich zunächst in geschlossener Stellung. Das Ventil 48 wird über eine Bedieneinrichtung (nicht gezeigt), wie ein Hebel, ein Schalter, eine Taste, eine Fußtaste oder einen Abzug gedreht und dadurch die Durchführung 49 in den Kanal 50 gedreht. Das Ventil 48 ist dann offen und der Kanal 50 erstreckt sich mit der Durchführung 49 von der Gaspatrone 44 bis zur Mündung des Ausströmkanals 51 aus der Vorrichtung 40 heraus.

Das Gas aus der Gaspatrone 44 kann dann also den gesamten Kanal 50 durchströmen. Im Bereich der Venturi-Düse 53 wird die Strömungsgeschwindigkeit des Gases erhöht. Durch die Bernoulli'schen Gesetze wird dadurch in der senkrecht auf den Kanal 50 treffenden Zuleitung des Vakuumanschlusses 52 ein Unterdruck erzeugt. Das Rückschlagventil 54 in der Zuleitung öffnet sich und der Unterdruck liegt am Vakuumanschluss 52 an. Dieser Unterdruck wird über eine Leitung in das Vakuumzementiersystem weitergeleitet, so dass dort ein Zement unter Unterdruck gemischt werden kann. Das Gas strömt dabei über den Schalldämpfer 55 geräuscharm aus der Vorrichtung 40 heraus in die Umgebung. Wenn der Gasstrom aus der Gaspatrone 44 endet, schließt das Rückschlagventil 54, so dass im Vakuumzementiersystem weiter unter Unterdruck beziehungsweise im Vakuum gemischt werden kann.

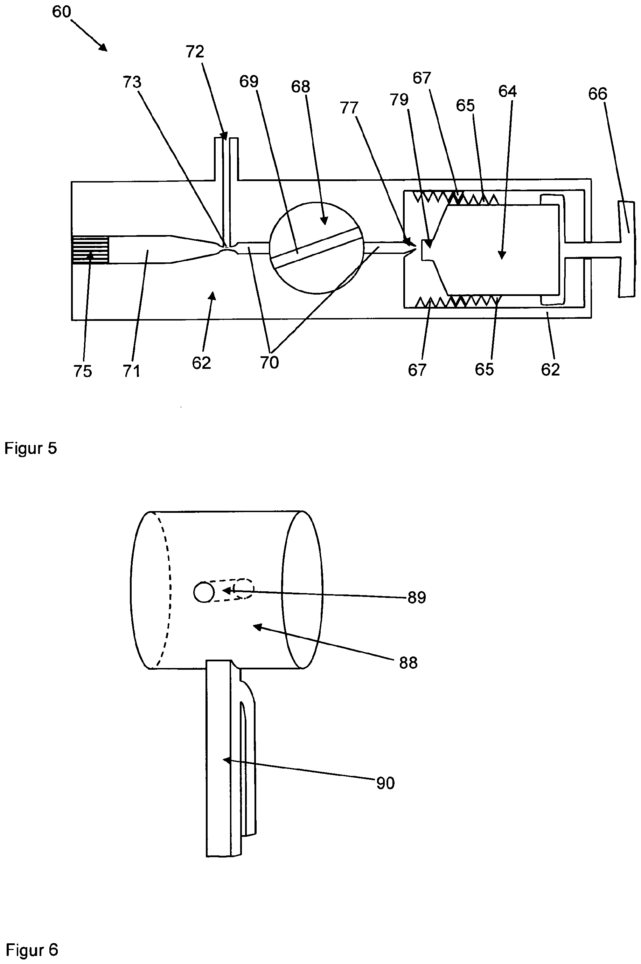

Figur 5 zeigt eine weitere schematische Querschnittansicht einer weiteren erfindungsgemäßen Vorrichtung 60, die ähnlich der Vorrichtung 40 nach Figur 4 aufgebaut ist. In einem Gehäuse 62 ist eine in Längsrichtung (in Figur 5 von rechts nach links) der Vorrichtung 60 beweglich gelagerte Gaspatrone 64 angeordnet. Die Gaspatrone 64 ist in einem Hohlraum im hinteren Ende der Vorrichtung 60 angeordnet. In diesem Hohlraum ist ein Innengewinde 67 vorgesehen, das in ein Außengewinde 65 der Gaspatrone 64 greift. An der Gaspatrone 64 ist ein Drehgriff 66 befestigt, mit dem die Gaspatrone 64 in das Außengewinde 67 eindrehbar ist und dadurch in Längsrichtung verschoben wird.

Im Inneren der Vorrichtung 60 ist ein drehbar gelagertes Ventil 68 mit einer Durchführung 69 angeordnet. In der gezeigten geschlossenen Stellung des Ventils 68 dichtet dieses einen Kanal 70 ab, der sich von dem Hohlraum am hinteren Ende der Vorrichtung 60 bis zu einem Auslass am vorderen Ende der Vorrichtung 60 erstreckt. Im vorderen Bereich der Vorrichtung ist der Kanal 70 geweitet und bildet dort einen Auslasskanal 71 mit größerem Durchmesser. Durch Drehen des Ventils 68 kann eine durchgehende Verbindung über die Kanäle 70, 71 und die Durchführung 69 bereitgestellt werden und das Ventil 68 ist geöffnet.

Auf der Oberseite der Vorrichtung 60 ist ein Vakuumanschluss 72 angeordnet, der eine Zuleitung umfasst. Diese ist über ein T-Stück 73 mit dem Kanal 70 verbunden. Im Bereich des T-Stücks 73 weist der Kanal 70 einen kleineren Durchmesser auf, als im Rest der Vorrichtung 60. Hierdurch wird eine effiziente Venturi-Düse 73 gebildet, so dass ein durch den Kanal 70 und die Durchführung 69 strömendes Gas am Vakuumanschluss 72 einen Unterdruck beziehungsweise ein Vakuum erzeugt. An dem Vakuumanschluss 72 kann ein Vakuumzementiersystem zum Mischen eines medizinischen Zements angeordnet sein oder angeordnet werden.

Im Ausströmkanal 71 ist im Bereich der Öffnung ein Schalldämpfer 75 angeordnet, der die durch das strömende Gas verursachten Geräusche dämpfen soll. Am anderen Ende des Kanals 70 ist ein Hohldorn 77 angeordnet, der die Gaspatrone 64 an deren Kopf 79 aufsticht, wenn diese mit dem Drehgriff 66 auf den Hohldorn 77 geschraubt wird. Die Durchführung der Stange des Drehgriffs 66 in den Hohlraum für die Gaspatrone 64 kann mit einer Dichtung abgedichtet sein. Zur Abdichtung der Verbindung von der Gaspatrone 64 über den Hohldorn 77 in den Kanal 70 kann um den Hohldorn 77 herum auf der Fläche des Hohlraums, aus dem der Hohldorn 77 herausragt, eine Dichtung angeordnet sein.

Die Vorrichtung 60 kann wie die Vorrichtung 40 nach Figur 4 verwendet werden, wobei die Vorrichtung 60 kein Rückschlagventil in der Zuleitung des Vakuumanschlusses 72 umfasst, so dass dieses auch nicht geöffnet werden muss. Das Ventil 68 kann, wie das Ventil 48 nach Figur 4 auch, über eine Taste, einen Hebel, einen Abzug oder eine andere Bedieneinrichtung bedienbar sein und gedreht werden. Der Unterdruck wird hier nur solange aufrechterhalten, solange noch Gas aus der Gaspatrone 64 strömt und solange das Ventil 68 in der geöffneten Stellung steht.

Die Vorrichtungen 40 und 60 können sowohl mit der Hand, als auch mit dem Fuß bedienbar sein. Dazu können sie dazu ausgelegt sein, in der Hand gehalten oder auf den Boden gelegt zu werden.

Figur 6 zeigt eine schematische perspektivische Ansicht eines Drehventils 88 mit einer Durchführung 89 und einem Abzug 90, wie es beispielsweise in einer erfindungsgemäßen Vorrichtung 1 nach Figur 1 verwendet werden kann. Grundsätzlich kann ein solches Drehventil 88 aber auch in den erfindungsgemäßen Vorrichtungen 40, 60 nach den Figuren 4 und 5 verwendet werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 20, 40, 60: Vorrichtung
- 2, 22, 42, 62: Gehäuse
- 6: Schraubdeckel
- 8, 28, 48, 68, 88: Ventil
- 10, 90: Abzug
- 12, 32, 52, 72: Vakuumanschluss
- 14: Griff
- 16, 36: Gasauslass
- 26, 66: Drehgriff
- 30: Fußtaste
- 31: Rückstellelement / Blattfeder
- 44, 64: Gaspatrone
- 45, 65: Außengewinde
- 47, 67: Innengewinde
- 49, 69, 89: Durchführung
- 50, 70: Kanal
- 51, 71: Ausströmkanal
- 53, 73: T-Stück / Venturi-Düse / Laval-Düse
- 54: Rückschlagventil
- 55, 75: Schalldämpfer
- 57, 77: Hohldorn
- 59, 79: Kopf der Gaspatrone

## Patentansprüche

1. Vorrichtung (1, 20, 40, 60) zum Erzeugen eines Vakuums für eine Vakuumzementiervorrichtung, die Vorrichtung (1, 20, 40, 60) aufweisend eine Gaspatrone (44, 64), einen Kanal (50, 70) und einen Anschluss für die Gaspatrone (44, 64), **dadurch gekennzeichnet, dass** der Anschluss eine Öffnungseinrichtung (57, 77) zum Öffnen der Gaspatrone (44, 64) umfasst und die Gaspatrone (44, 64) über den Anschluss mit dem Kanal (50, 70) druckdicht verbindbar oder verbunden ist, so dass ein Gas aus der geöffneten Gaspatrone (44, 64) entlang des Kanals (50, 70) strömt, wobei der Kanal (50, 70) den Anschluss mit der Umgebung der Vorrichtung (1, 20, 40, 60) verbindet und der Kanal (50, 70) zumindest ein T-Stück (53, 73) mit einem Vakuumanschluss (12, 32, 52, 72) für die Vakuumzentiervorrichtung umfasst, wobei das T-Stück (53, 73) als Venturi-Düse (53, 73) ausgebildet ist, so dass das durch den Kanal (50, 70) strömende Gas aus der geöffneten Gaspatrone (44, 64) am Vakuumanschluss (12, 32, 52, 72) einen Unterdruck erzeugt, wobei die Venturi-Düse (53, 73) in einen Ausströmkanal (51, 71) mit einem vergrößerten Durchmesser mündet, der gasdurchlässig mit der Umgebung der Vorrichtung (1, 20, 40, 60) verbunden ist.

2. Vorrichtung (1, 20, 40, 60) nach Anspruch 1, **dadurch gekennzeichnet, dass**
im Kanal (50, 70) zumindest ein bedienbares Absperrelement (8, 28, 48, 68, 88) angeordnet ist, mit dem der Gasstrom aus der geöffneten Gaspatrone (44, 64) durch den Kanal (50, 70) unterbrechbar und/oder einstellbar ist, wobei insbesondere das bedienbare Absperrelement (8, 28, 48, 68, 88) ein Rückstellelement (31) umfasst, das vorzugsweise ein Federelement (31) umfasst, besonders bevorzugt ein Federelement (31) aus Metall.

3. Vorrichtung (1, 20, 40, 60) nach Anspruch 2, **dadurch gekennzeichnet, dass** das Absperrelement (8, 28, 48, 68, 88) ein Ventil (8, 28, 48, 68, 88) oder die Absperrelemente (8, 28, 48, 68, 88) Ventile (8, 28, 48, 68, 88) sind, vorzugsweise zumindest ein Drehventil (8, 28, 48, 68, 88), ein Kugelventil, ein Quetschventil, ein Stiftventil und/oder ein Schieber.

4. Vorrichtung (1, 20, 40, 60) nach Anspruch 3, **dadurch gekennzeichnet, dass**
das Ventil (8, 28, 48, 68, 88) ein konisch geformter, zumindest bereichsweise rotationssymmetrischer Körper ist, der in einer zur Form des Ventils (8, 28, 48, 68, 88) passenden konischen Öffnung in der Vorrichtung (1, 20, 40, 60) drehbar gelagert ist, wobei die Außenwände des Ventils (8, 28, 48, 68, 88) bis auf die Öffnungen des Kanals (50, 70) dicht mit den Wänden der konischen Öffnung in der Vorrichtung (1, 20, 40, 60) in jeder Position des Ventils (8, 28, 48, 68, 88) abschließen, so dass die Verbindungen zumindest zu dem Kanal (50, 70) dicht sind.

5. Vorrichtung (1, 20, 40, 60) nach einem der vorangehenden Ansprüche, **dadurch gekennzeichnet, dass**
der Anschluss ein Innengewinde (47, 67) zur Aufnahme einer Gaspatrone (44, 64) mit einem Außengewinde (45, 65) umfasst.

6. Vorrichtung (1, 20, 40, 60) nach einem der vorangehenden Ansprüche, **dadurch gekennzeichnet, dass**
der Anschluss ein Dichtungsmittel, vorzugsweise eine Gummidichtung zum Abdichten der Verbindung der Gaspatrone (44, 64) mit dem Kanal (50, 70) umfasst.

7. Vorrichtung (1, 20, 40, 60) nach einem der vorangehenden Ansprüche, **dadurch gekennzeichnet, dass**
die Öffnungseinrichtung (57, 77) einen Dorn (57, 77) umfasst, vorzugsweise einen Hohldorn (57, 77), der ein Ende des Kanals (50, 70) bildet.

8. Vorrichtung (1, 20, 40, 60) nach Anspruch 7, **dadurch gekennzeichnet, dass**
die Öffnungseinrichtung (57, 77) eine bedienbare Presseinrichtung (6, 26, 66) umfasst, mit der die Gaspatrone (44, 64) auf den Dorn (57, 77) pressbar ist, insbesondere im Bereich einer Membran als Sollbruchstelle der Gaspatrone (44, 64), wobei die Gaspatrone (44, 64) dadurch zu öffnen ist und wobei vorzugsweise die Presseinrichtung (6, 26, 66) drehbar ist.

9. Vorrichtung (1, 20, 40, 60) nach einem der vorangehenden Ansprüche, **dadurch gekennzeichnet, dass**
die Gaspatrone (44, 64) derart im Bereich der Öffnungseinrichtung (57, 77) in die Vorrichtung (1, 20, 40, 60) eingebaut ist, dass sie durch ein Bedienen der Öffnungseinrichtung (57, 77) zu öffnen ist, wobei die Gaspatrone (44, 64) vorzugsweise ein Außengewinde (45, 65) umfasst, das in ein Innengewinde (47, 67) greift.

10. Vorrichtung (1, 20, 40, 60) nach einem der vorangehenden Ansprüche, **dadurch gekennzeichnet, dass**
die Venturi-Düse (53, 73) durch eine Verjüngung des Kanals (50, 70) gebildet ist und der Vakuumanschluss (12, 32, 52, 72) im Bereich dieser Verjüngung mit dem Kanal (50, 70) verbunden ist, wobei vorzugsweise eine Laval-Düse gebildet ist.

11. Vorrichtung (1, 20, 40, 60) nach einem der vorangehenden Ansprüche, **dadurch gekennzeichnet, dass**
die Vorrichtung (1, 20, 40, 60) zumindest eine Bedieneinrichtung (6, 10, 26, 30, 66, 90) umfasst, mit der die Öffnungseinrichtung (57, 77) und/oder das Absperrelement (8, 28, 48, 68, 88) bedienbar ist oder sind, wobei die Bedieneinrichtung (6, 10, 26, 30, 66, 90) als Abzug (10, 90), als Taste (30) oder als Pedal ausgebildet ist und wobei die Bedieneinrichtung (6, 10, 26, 30, 66, 90) vorzugsweise eine Rückstelleinrichtung (31), insbesondere mit einer Metallfeder umfasst.

12. Vorrichtung (1, 20, 40, 60) nach einem der vorangehenden Ansprüche, **dadurch gekennzeichnet, dass**
an der Unterseite der Vorrichtung (1, 20, 40, 60) ein Griff (14) zum Halten der Vorrichtung (1, 20, 40, 60) angeordnet ist, wobei vorzugsweise die Bedieneinrichtung (10, 30, 90) ein Abzug (10, 90) oder eine Taste (30) im Bereich des Griffs (14) der Vorrichtung (1, 20, 40, 60) ist, so dass die Vorrichtung (1, 20, 40, 60) einhändig bedienbar ist.

13. Vorrichtung (1, 20, 40, 60) nach einem Ansprüche 1 bis 11, **dadurch gekennzeichnet, dass**
die Vorrichtung (1, 20, 40, 60) mit einem ebenen Boden zum Aufstellen auf einem ebenen Untergrund ausgebildet ist, wobei vorzugsweise die Bedieneinrichtung (10, 30, 90) ein Pedal oder eine Taste (30) auf der Oberseite der Vorrichtung (1, 20, 40, 60) ist.

14. Vorrichtung (1, 20, 40, 60) nach einem der vorangehenden Ansprüche, **dadurch gekennzeichnet, dass**
die Vorrichtung (1, 20, 40, 60) im Wesentlichen aus Kunststoff besteht, vorzugsweise bis auf die Öffnungseinrichtung (57, 77), die Gaspatrone (44, 64), das Rückschlagventil (54) und/oder das Rückstellelement (31) oder die Rückstellelemente (31) aus Kunststoff besteht.

15. Vorrichtung (1, 20, 40, 60) nach einem der vorangehenden Ansprüche, **dadurch gekennzeichnet, dass**
die Vorrichtung (1, 20, 40, 60) eine Masse von weniger als 10 kg hat, vorzugsweise zwischen 0,1 kg und 5 kg, besonders bevorzugt zwischen 0,5 kg und 3 kg.

16. Vorrichtung (1, 20, 40, 60) nach einem der vorangehenden Ansprüche, **dadurch gekennzeichnet, dass**
der Kanal (50, 70) an der dünnsten Stelle eine Querschnittsfläche von weniger als 10 mm² hat, insbesondere zwischen 0,1 mm² und 5 mm² und/oder der Kanal (50, 70) einen schmalen Spalt mit einer Breite von weniger als 1 mm bildet oder eine schlanke Röhre mit einem Durchmesser von weniger als 1 mm ist.

17. Vorrichtung (1, 20, 40, 60) nach einem der vorangehenden Ansprüche, **dadurch gekennzeichnet, dass**
die Geometrie und der Strömungswiderstand des Kanals (50, 70) derart an die Kapazität der Gaspatrone (44, 64) angepasst sind, dass das Gas aus der geöffneten Gaspatrone (44, 64) zumindest 5 s aus der Gaspatrone (44, 64) durch den Kanal (50, 70) strömen kann, bevor die Gaspatrone (44, 64) kein komprimiertes Gas mehr enthält, vorzugsweise zwischen 10 s und 100 s, besonders bevorzugt zwischen 20 s und 60 s, ganz besonders bevorzugt zwischen 25 s und 45 s.

18. Vorrichtung (1, 20, 40, 60) nach einem der vorangehenden Ansprüche, **dadurch gekennzeichnet, dass**
im Vakuumanschluss (12, 32, 52, 72) ein Rückschlagventil (54) angeordnet ist, wobei das Rückschlagventil (54) durch den durch die Venturi-Düse (53, 73) erzeugten Unterdruck zu öffnen ist und den Vakuumanschluss (12, 32, 52, 72) verschließt, wenn kein oder sehr wenig Gas aus der geöffneten Gaspatrone (44, 64) durch den Kanal (50, 70) strömt und wobei vorzugsweise das Rückschlagventil (54) ein Rückstellelement umfasst.

19. Vorrichtung (1, 20, 40, 60) nach einem der vorangehenden Ansprüche, **dadurch gekennzeichnet, dass**
im Ausströmkanal (51, 71) ein Schalldämpfer (55, 75) angeordnet ist, besonders bevorzugt im Bereich der Mündung zur Umgebung der Vorrichtung (1, 20, 40, 60).

20. Vorrichtung (1, 20, 40, 60) nach einem der vorangehenden Ansprüche, **dadurch gekennzeichnet, dass**
die Vorrichtung (1, 20, 40, 60) über den Vakuumanschluss (12, 32, 52, 72) an ein Vakuumzementiersystem zur Herstellung eines medizinischen Knochenzements angeschlossen ist oder anschließbar ist.

21. Verfahren zum Erzeugen eines Unterdrucks in einem Vakuumzementiersystem mit einer Vorrichtung (1, 20, 40, 60) nach einem der vorangehenden Ansprüche, **gekennzeichnet durch**
Öffnen einer Gaspatrone (44, 64),
Durchströmen des Gases aus der Gaspatrone (44, 64) durch eine Venturi-Düse (53, 73), wodurch der Unterdruck erzeugt wird und
Anlegen des Unterdrucks an das Vakuumzementiersystem.

22. Verfahren nach Anspruch 20, **dadurch gekennzeichnet, dass**
nach dem Öffnen der Gaspatrone (44, 64) das Absperrelement (8, 28, 48, 68, 88) geöffnet wird und nach dem Öffnen des Absperrelements (8, 28, 48, 68, 88) das Gas durch die Venturi-Düse (53, 73) strömt.

23. Verfahren nach einem der Ansprüche 20 oder 21, **dadurch gekennzeichnet, dass**
ein medizinscher Zement, insbesondere ein medizinischer Knochenzement unter Einwirkung des Unterdrucks im Vakuumzementiersystem gemischt wird.

## Claims

1. Device (1, 20, 40, 60) for generating a vacuum for a vacuum cementing device, the device (1, 20, 40, 60) comprising a gas cartridge (44, 64), a channel (50, 70) and a connector for the gas cartridge (44, 64), **characterised in that**
the connector comprises an opening facility (57, 77) for opening the gas cartridge (44, 64), and the gas cartridge (44, 64) is connectable or is connected through the connector to the channel (50, 70) in a pressure-tight manner such that a gas from the opened gas cartridge (44, 64) flows along the channel (50, 70), whereby the channel (50, 70) connects the connector to the surroundings of the device (1, 20, 40, 60) and the channel (50, 70) comprises at least one T-piece (53, 73) having a vacuum connector (12, 32, 52, 72) for the vacuum cementing device, whereby the T-piece (53, 73) is provided as a Venturi nozzle (53, 73) such that the gas from the opened gas cartridge (44, 64) flowing through the channel (50, 70) generates a negative pressure at the vacuum connector (12, 32, 52, 72), wherein the Venturi nozzle (53, 73) merges into an outflow channel (51, 71) having a larger diameter that is connected to the surroundings of the device (1, 20, 40, 60) in a gas-permeable manner.

2. Device (1, 20, 40, 60) according to claim 1, **characterised in that**
at least one operable lock element (8, 28, 48, 68, 88) is arranged in the channel (50, 70), which makes the gas flow from the opened gas cartridge (44, 64) through the channel (50, 70) interruptible and/or adjustable, whereby, in particular, the operable lock element (8, 28, 48, 68, 88) comprises a restoring element (31) which preferably comprises a spring element (31), particularly preferably a spring element (31) made of metal.

3. Device (1, 20, 40, 60) according to claim 2, **characterised in that**
the lock element (8, 28, 48, 68, 88) is a valve (8, 28, 48, 68, 88) or the lock elements (8, 28, 48, 68, 88) are valves (8, 28, 48, 68, 88), preferably at least one rotary valve (8, 28, 48, 68, 88), one ball valve, one pinch valve, one pin valve and/or one slider.

4. Device (1, 20, 40, 60) according to claim 3, **characterised in that**
the valve (8, 28, 48, 68, 88) is a conical, at least partially rotationally symmetrical body that is supported as in a bearing in a matching conical opening in the device (1, 20, 40, 60) such that it can be rotated, whereby the external walls of the valve (8, 28, 48, 68, 88) are tight against the walls of the conical opening in the device (1, 20, 40, 60), except for the openings of the channel (50, 70), in each position of the valve (8, 28, 48, 68, 88) such that the connections are tight at least with respect to the channel (50, 70).

5. Device (1, 20, 40, 60) according to any one of the preceding claims, **characterised in that**
the connector comprises an internal thread (47, 67) for taking up a gas cartridge (44, 64) fitted with an external thread (45, 65).

6. Device (1, 20, 40, 60) according to any one of the preceding claims, **characterised in that**
the connector comprises a sealing means, preferably a rubber gasket, for sealing the connection of the gas cartridge (44, 64) with respect to the channel (50, 70).

7. Device (1, 20, 40, 60) according to any one of the preceding claims, **characterised in that**
the opening facility (57, 77) comprises a mandrel (57, 77), preferably a hollow mandrel (57, 77), that forms one end of the channel (50, 70).

8. Device (1, 20, 40, 60) according to claim 7, **characterised in that**
the opening facility (57, 77) comprises an operable pressure-exerting facility (6, 26, 66) through which the gas cartridge (44, 64) is pressable onto the mandrel (57, 77), in particular in the area of a membrane serving as predetermined breaking point of the gas cartridge (44, 64), whereby the gas cartridge (44, 64) is thus openable and whereby the pressure-exerting facility (6, 26, 66) preferably is rotatable.

9. Device (1, 20, 40, 60) according to any one of the preceding claims, **characterised in that**
the gas cartridge (44, 64) is incorporated in the device (1, 20, 40, 60) in the area of the opening facility (57, 77) in such manner that it is openable by operating the opening facility (57, 77), whereby the gas cartridge (44, 64) preferably comprises an external thread (45, 65) that engages an internal thread (47, 67).

10. Device (1, 20, 40, 60) according to any one of the preceding claims, **characterised in that**
the Venturi nozzle (53, 73) is formed by a tapering of the channel (50, 70), and the vacuum connector (12, 32, 52, 72) is connected to the channel (50, 70) in the area of said tapering, wherein preferably a Laval nozzle is formed.

11. Device (1, 20, 40, 60) according to any one of the preceding claims, **characterised in that**
the device (1, 20, 40, 60) comprises at least one operating facility (6, 10, 26, 30, 66, 90) by which the opening facility (57, 77) and/or the lock element (8, 28, 48, 68, 88) is or are operable, whereby the operating facility (6, 10, 26, 30, 66, 90) is provided as trigger (10, 90), button (30) or pedal, and whereby the operating facility (6, 10, 26, 30, 66, 90) preferably comprises a restoring facility (31), in particular having a metal spring.

12. Device (1, 20, 40, 60) according to any one of the preceding claims, **characterised in that**
a handle (14) for holding the device (1, 20, 40, 60) is arranged on the underside of the device (1, 20, 40, 60), whereby preferably the operating facility (10, 30, 90) is a trigger (10, 90) or a button (30) in the area of the handle (14) of the device such that the device (1, 20, 40, 60) is operable single handed.

13. Device (1, 20, 40, 60) according to any one of the claims 1 to 11, **characterised in that**
the device (1, 20, 40, 60) is provided with a level floor for set-up on a level underground, whereby preferably the operating facility (10, 30, 90) is a pedal or a button (30) on the upper side of the device (1, 20, 40, 60).

14. Device (1, 20, 40, 60) according to any one of the preceding claims, **characterised in that**
the device (1, 20, 40, 60) consists essentially of plastic material, preferably consists of plastic material with the exception of the opening facility (57, 77), the gas cartridge (44, 64), the non-return valve (54) and/or the restoring element (31) or the restoring elements (31).

15. Device (1, 20, 40, 60) according to any one of the preceding claims, **characterised in that**
the mass of the device (1, 20, 40, 60) is less than 10 kg, preferably between 0.1 kg and 5 kg, particularly preferably between 0.5 kg and 3 kg.

16. Device (1, 20, 40, 60) according to any one of the preceding claims, **characterised in that**
the cross-sectional area of the channel (50, 70) at its thinnest section is less than 10 mm², in particularly between 0.1 mm² and 5 mm² and/or **in that** the channel (50, 70) forms a narrow gap of a width of less than 1 mm or is a slender tube with a diameter of less than 1 mm.

17. Device (1, 20, 40, 60) according to any one of the preceding claims, **characterised in that**
the geometry and the flow resistance of the channel (50, 70) are matched to the capacity of the gas cartridge (44, 64) in such manner that the gas from the opened gas cartridge (44, 64) can flow for at least 5 s from the gas cartridge (44, 64) through the channel (50, 70) before the gas cartridge (44, 64) no longer contains any compressed gas, preferably between 10 s and 100 s, more preferably between 20 s and 60 s, and particularly preferably between 25 s and 45 s.

18. Device (1, 20, 40, 60) according to any one of the preceding claims, **characterised in that**
a non-return valve (54) is arranged in the vacuum connector (12, 32, 52, 72), whereby the non-return valve (54) is openable by the negative pressure generated through the Venturi nozzle (53, 73) and then closes the vacuum connector (12, 32, 52, 72) when no or only very little gas flows from the opened gas cartridge (44, 64) through the channel (50, 70), and whereby the non-return valve (54) preferably comprises a restoring element.

19. Device (1, 20, 40, 60) according to any one of the preceding claims, **characterised in that**
a silencer (55, 75) is arranged in the outflow channel (51, 71), particularly preferably in the region of the merging site to the surroundings of the device (1, 20, 40, 60).

20. Device (1, 20, 40, 60) according to any one of the preceding claims, **characterised in that**
the device (1, 20, 40, 60) is connected or connectable through the vacuum connector (12, 32, 52, 72) to a vacuum cementing system for producing a medical bone cement.

21. Method for generating a negative pressure in a vacuum cementing system, using a device (1, 20, 40, 60) according to any one of the preceding claims, **characterised in** opening a gas cartridge (44, 64);
the gas from the gas cartridge (44, 64) flowing through a Venturi nozzle (53, 73) by means of which the negative pressure is generated; and
applying the negative pressure to the vacuum cementing system.

22. Method according to claim 20, **characterised in that**
the lock element (8, 28, 48, 68, 88) is opened after the gas cartridge (44, 64) is opened, and the gas flows through the Venturi nozzle (53, 73) after the lock element (8, 28, 48, 68, 88) is opened.

23. Method according to any one of the claims 20 or 21, **characterised in that**
a medical cement, in particular a medical bone cement, is being mixed under the action of the negative pressure in the vacuum cementing system.

## Revendications

1. Dispositif (1, 20, 40, 60) de génération d'un vide pour un dispositif de cimentation sous vide, le dispositif (1, 20, 40, 60) présentant une cartouche de gaz (44, 64), un canal (50, 70) et un connecteur pour la cartouche de gaz (44, 64),
**caractérisé en ce que**
le connecteur comprend un dispositif d'ouverture (57, 77) pour l'ouverture de la cartouche de gaz (44, 64) et la cartouche de gaz (44, 64) peut être reliée ou est reliée avec le canal (50, 70) de manière étanche à la pression par le biais du connecteur, de sorte qu'un gaz s'écoule à partir de la cartouche de gaz (44, 64) ouverte le long du canal (50, 70), où le canal (50, 70) relie le connecteur avec l'environnement du dispositif (1, 20, 40, 60) et le canal (50, 70) comprend au moins une pièce en T (53, 73) avec un connecteur destiné au vide (12, 32, 52, 72) pour le dispositif de cimentation sous vide, où la pièce en T (53, 73) est conçue sous la forme d'une buse à effet Venturi (53, 73) de sorte que le gaz s'écoulant à travers le canal (50, 70) à partir de la cartouche à gaz (44, 64) ouverte génère une pression négative au niveau du connecteur pour le vide (12, 32, 52, 722), où la buse à effet Venturi (53, 73) débouche dans un canal de sortie (51, 71) avec un diamètre agrandi, qui est relié avec l'environnement du dispositif (1,20, 40, 60) en étant perméable aux gaz.

2. Dispositif (1, 20, 40, 60) selon la revendication 1, **caractérisé en ce que**,
dans le canal (50, 70), au moins un élément de blocage (8, 28, 48, 68, 88) manoeuvrable est disposé, avec lequel le flux de gaz sortant de la cartouche de gaz (44, 64) ouverte peut être interrompu et/ou être réglé par le canal (50, 70), où, en particulier, l'élément de blocage (8, 28, 48, 68, 88) manoeuvrable comprend un élément de retour (31), qui comprend de préférence un élément ressort (31), notamment, de préférence un élément ressort en métal.

3. Dispositif (1, 20, 40, 60) selon la revendication 2, **caractérisé en ce que**
l'élément de blocage (8, 28, 48, 68, 88) est une vanne (8, 28, 48, 68, 88) ou les éléments de blocage (8, 28, 48, 68, 88) sont des vannes (8, 28, 48, 68, 88), de préférence, au moins une soupape rotative (8, 28, 48, 68, 88), une vanne à boisseau sphérique, une vanne à striction, une vanne à poussoir et/ou un poussoir.

4. Dispositif (1, 20, 40, 60) selon la revendication 3, **caractérisé en ce que**
la vanne (8, 28, 48, 68, 88) est un corps de forme conique, symétrique en rotation au moins par endroits, qui est logé en pouvant être mis en rotation dans une ouverture conique adaptée à la forme de la vanne (8, 28, 48, 68, 88) dans le dispositif (1, 20, 40, 60), où les parois extérieures de la vanne (8, 28, 48, 68, 88) ferment de manière étanche jusqu'aux ouvertures du canal (50, 70) avec les parois de l'ouverture conique dans le dispositif (1, 20, 40, 60) dans toutes les positions de la vanne (8, 28, 48, 68, 88) de sorte que les liaisons sont étanches au moins vers le canal (50, 70).

5. Dispositif (1, 20, 40, 60) selon l'une des revendications précédentes, **caractérisé en ce que**
le connecteur comprend un filetage intérieur (47, 67) pour l'admission d'une cartouche de gaz (44, 64) avec un filetage extérieur (45, 65).

6. Dispositif (1, 20, 40, 60) selon l'une des revendications précédentes, **caractérisé en ce que**
le connecteur comprend un moyen assurant l'étanchéité, de préférence, un joint en caoutchouc pour rendre étanche la liaison de la cartouche de gaz (44, 64) avec le canal (50, 70).

7. Dispositif (1, 20, 40, 60) selon l'une des revendications précédentes, **caractérisé en ce que**
le dispositif d'ouverture (57, 77) comprend un mandrin (57, 77), de préférence, un mandrin creux (57, 77) qui forme une extrémité du canal (50, 70).

8. Dispositif (1, 20, 40, 60) selon la revendication 7, **caractérisé en ce que**
le dispositif d'ouverture (57, 77) comprend un dispositif de pressage (6, 26, 66) manoeuvrable avec lequel la cartouche de gaz (44, 64) peut être pressée sur le mandrin (57, 77), notamment dans la région d'une membrane sous la forme d'un point de rupture souhaité de la cartouche de gaz (44, 64), où la cartouche de gaz (44, 64) peut ainsi s'ouvrir et où, de préférence, le dispositif de presse (6, 26, 66) peut être mis en rotation.

9. Dispositif (1, 20, 40, 60) selon l'une des revendications précédentes, **caractérisé en ce que**
la cartouche de gaz (44, 64) est montée dans la région du dispositif d'ouverture (57, 77) dans le dispositif (1, 20, 40, 60) de telle manière qu'elle peut être ouverte par une manoeuvre du dispositif d'ouverture (57, 77), où la cartouche de gaz (44, 64) comprend de préférence un filetage extérieur (45, 65) qui se met en prise dans un filetage intérieur (47, 67).

10. Dispositif (1, 20, 40, 60) selon l'une des revendications précédentes, **caractérisé en ce que**
la buse à effet Venturi (53, 73) est formée par un amenuisement du canal (50, 70) et la connexion pour le vide (12, 32, 52, 72) est reliée dans la région de cet amenuisement avec le canal (50, 70), où, de préférence, une tuyère de Laval est formée.

11. Dispositif (1, 20, 40, 60) selon l'une des revendications précédentes, **caractérisé en ce que**
le dispositif (1, 20, 40, 60) comprend au moins un dispositif de manoeuvre (6, 10, 26, 30, 66, 90) avec lequel le dispositif d'ouverture (57, 77) et/ou l'élément de blocage (8, 28, 48, 68, 88) peuvent être manoeuvrés, où le dispositif de manoeuvre (6, 10, 26, 30, 66, 90) est conçu sous la forme d'un retrait (10, 90), sous la forme d'une touche (30), ou sous la forme d'une pédale et où le dispositif de manoeuvre (6, 10, 26, 30, 66, 90) comprend de préférence un dispositif de retour (31), notamment avec un ressort métallique.

12. Dispositif (1, 20, 40, 60) selon l'une des revendications précédentes, **caractérisé en ce**
**qu'**une poignée (14) est disposée sur la face inférieure du dispositif (1, 20, 40, 60) pour porter le dispositif (1, 20, 40, 60), où de préférence le dispositif de manoeuvre (10, 30, 90) est un retrait (10, 90) ou une touche (30) dans la région de la poignée (14) du dispositif (1, 20, 40, 60) de sorte que le dispositif (1, 20, 40, 60) peut être manoeuvré par une main.

13. Dispositif (1, 20, 40, 60) selon l'une des revendications 1 à 11, **caractérisé en ce que**
le dispositif (1, 20, 40, 60) est conçu avec un socle plat pour la mise en position debout sur une base plane, où de préférence le dispositif de manoeuvre (10, 30, 90) est une pédale ou une touche (30) sur la face supérieure du dispositif (1, 20, 40, 60).

14. Dispositif (1, 20, 40, 60) selon l'une des revendications précédentes, **caractérisé en ce que**
le dispositif (1, 20, 40, 60) est constitué essentiellement de matière plastique, de préférence y compris le dispositif d'ouverture (57, 77), la cartouche de gaz (44, 64), le clapet anti-retour (54) et/ou l'élément de retour (31) ou les éléments de retour (31) sont constitués de matière plastique.

15. Dispositif (1, 20, 40, 60) selon l'une des revendications précédentes, **caractérisé en ce que**
le dispositif (1, 20, 40, 60) a une masse inférieure à 10 kg, de préférence entre 0,1 kg et 5 kg, de manière particulièrement préférée entre 0,5 kg et 3 kg.

16. Dispositif (1, 20, 40, 60) selon l'une des revendications précédentes, **caractérisé en ce que**
le canal (50, 70) au niveau du point le plus étroit a une surface de section transversale inférieure à 10 mm², notamment entre 0,1 mm² et 5 mm² et/ou le canal (50, 70) forme une fente avec une largeur inférieure à 1 mm ou est un tube fin avec un diamètre inférieur à 1 mm.

17. Dispositif (1, 20, 40, 60) selon l'une des revendications précédentes, **caractérisé en ce que**
la géométrie et la résistance à l'écoulement du canal (50, 70) sont adaptées de telle manière à la capacité de la cartouche de gaz (44, 64) que le gaz peut s'écouler à partir de la cartouche de gaz pendant au moins 5 s en sortant de la cartouche de gaz (44, 64) à travers le canal (50, 70) avant que la cartouche à gaz (44, 64) ne contienne plus de gaz comprimé, de préférence entre 10 s et 100 s, de manière particulièrement préférée, entre 20 s et 60 s, idéalement, entre 25 s et 45 s.

18. Dispositif (1, 20, 40, 60) selon l'une des revendications précédentes, **caractérisé en ce**
**qu'**un clapet anti-retour (34) est disposé dans le connecteur pour le vide (12, 32, 52, 72), où le clapet anti-retour (54) peut être ouvert par la pression négative générée par la buse à effet Venturi (53, 73) et ferme le connecteur pour le vide (12, 32, 52, 72) lorsque plus ou très peu de gaz s'écoule à partir de la cartouche de gaz (44, 64) à travers le canal (50, 70) et où de préférence le clapet anti-retour (54) comprend un élément de retour.

19. Dispositif (1, 20, 40, 60) selon l'une des revendications précédentes, **caractérisé en ce**
**qu'**un atténuateur sonore (55, 75) est disposé dans le canal de sortie (51, 71), de manière particulièrement préférée dans la région de l'embouchure vers l'environnement du dispositif (1, 20, 40, 60).

20. Dispositif (1, 20, 40, 60) selon l'une des revendications précédentes, **caractérisé en ce que**
le dispositif (1, 20, 40, 60) est raccordé ou peut être raccordé à un système de cimentation sous vide pour la fabrication d'un ciment osseux médical par le biais du connecteur pour le vide (12, 32, 52, 72).

21. Procédé de génération d'une pression négative dans un système de cimentation sous vide avec un dispositif (1, 20, 40, 60) selon l'une des revendications précédentes, **caractérisé par**
l'ouverture de la cartouche de gaz (44, 64),
l'écoulement du gaz à partir de la cartouche de gaz (44, 64) par une buse à effet Venturi (53, 73), ce par quoi la pression négative est générée et
l'application de la pression négative au système de cimentation sous vide.

22. Procédé selon la revendication 20, **caractérisé en ce**
**qu'**après l'ouverture de la cartouche de gaz (44, 64), l'élément de blocage (8, 28, 48, 68, 88) est ouvert et après l'ouverture de l'élément de blocage (8, 28, 48, 68, 88), le gaz s'écoule à travers la buse à effet Venturi (53, 73).

23. Procédé selon l'une des revendications 20 ou 21, **caractérisé en ce**
**qu'**un ciment médical, notamment un ciment osseux médical est mélangé sous l'influence de la pression négative dans le système de cimentation sous vide.
